# DEMANDE DE BREVET EUROPEEN

(11) **EP 1 563 827 A2**
(43) Date de publication de la demande: **17.08.2005**
(21) Numéro de dépôt: 05290115.4
(22) Date de dépôt: 19.01.2005
(51) Int. Cl.: A61K 7/06, A61K 7/48

(54) **Procédé de préparation d'une composition de traitement cosmétique à partir de fluide sous pression, et d'actifs cosmétiques non colorants sensibles à un stimulus extérieur**

(30) Priorité: 29.01.2004 FR 0400852
(71) Demandeur: L'OREAL, 75008 Paris (FR)
(72) Inventeur: Grollier, Jean-Francois, 75006 Paris (FR); Dubief, Claude, 78150 Le Chesnay (FR); De La Mettrie, Roland, 78110 Le Vesinet (FR)
(74) Mandataire: Casalonga, Axel

(57) **Abrégé**

La présente invention concerne un procédé de préparation d'une composition de traitement cosmétique des matières kératiniques, par percolation de fluide comprenant au moins de la vapeur d'eau, sous une pression d'au moins 3 bars au travers d'au moins un actif cosmétique non colorant sensible à un stimulus extérieur, sous forme solide ou pâteuse.

L'invention concerne aussi un dispositif de conditionnement d'un tel actif ainsi que le procédé de traitement cosmétique mettant en oeuvre une telle composition.

## Description

La présente invention a pour objet un procédé de préparation d'une composition destinée au traitement cosmétique des matières kératiniques, en particulier de la peau ou des fibres kératiniques humaines telles que les cheveux, ainsi qu'un procédé de traitement cosmétique des matières kératiniques à partir de cette composition.

En cosmétique, on cherche toujours à améliorer les propriétés cosmétiques des matières kératiniques. Il est ainsi connu d'utiliser dans des compositions pour application topique des actifs cosmétiques permettant de lutter contre les agressions extérieures comme la pollution et le rayonnement ultraviolet, de lutter contre les dommages des fibres kératiniques ou de la peau, notamment les signes du vieillissement de la peau, d'hydrater et de nourrir les fibres kératiniques et la peau et d'apporter à la peau et aux autre matières kératiniques traitées par ces compositions, tous les bienfaits liés à ces actifs cosmétiques non colorants.

Par "actif cosmétique non colorant", on entend au sens de la présente invention tout actif qui, mis en contact avec les matières kératiniques, notamment avec la peau, les lèvres, les ongles ou les cheveux, n'apportent aucune coloration durable ou temporaire des matières kératiniques.

Par exemple, au cours du processus de vieillissement, il apparaît différents signes sur la peau humaine, très caractéristiques de ce vieillissement, se traduisant notamment par une modification de la structure et de la fonction cutanée. Les principaux signes cliniques de vieillissement cutané sont notamment les suivants : apparition de ridules puis de rides profondes en augmentation avec l'âge, et désorganisation du « grain » de la peau, c'est-à-dire que le micro-relief est moins régulier et présente un caractère anisotrope.

Par ailleurs, le teint de la peau est généralement modifié ; il apparaît plus pâle et plus jaune, ce qui semble être dû essentiellement à une désorganisation de la microcirculation (moins d'hémoglobine au niveau du derme papillaire). De nombreuses taches colorées apparaissent en surface, ce qui est dû à une mélanogénèse altérée. Un autre signe clinique de vieillissement est l'aspect sec et rêche de la peau qui est dû essentiellement à une desquamation plus importante, ces squames en diffractant les rayons lumineux participant aussi à l'aspect un peu gris du teint.

Ainsi, il est connu de traiter notamment les signes du vieillissement et les défauts de pigmentation, en utilisant des compositions cosmétiques ou dermatologiques contenant par exemple, des vitamines, des enzymes, de l'urée, la rutine, certains acides tels que décrits ci-dessous, et/ou des acides gras polyinsaturés.

Il est également connu de traiter des fibres kératiniques par des vitamines, des filtres solaires tels que des esters cinnamiques, des composés antioxydants ou antiradicalaires tels que des flavonoïdes, lorsque les fibres ont été soumises à un traitement de réduction, comme une permanente, et/ou d'oxydation, comme une coloration, ou qu'elles ont été soumises à un stimulus extérieur, comme la lumière. Grâce à ce traitement, on obtient un ravivement de la couleur des fibres, de leur brillance, et une amélioration du lissage des fibres.

Cependant, certains actifs cosmétiques non colorants présentent l'inconvénient d'être instables vis-à-vis de l'eau, d'agents oxydants et/ou de la lumière, à savoir ils subissent une dégradation par un mécanisme d'hydrolyse chimique, d'oxydation, de photolyse ou de photodégradation, ou encore d'échange d'ions, ce qui conduit à des compositions cosmétiques peu efficaces et/ou d'aspect, d'odeur et/ou toucher inacceptables pour le consommateur, en quelques jours.

Ces actifs cosmétiques non colorants présentant une telle instabilité seront dénommés ci-après "actifs cosmétiques non colorants sensibles à un stimulus extérieur", c'est-à-dire sensibles à l'eau, à l'oxydation, à la lumière et/ou à l'élévation de la température sur de longues périodes.

Ces derniers sont donc difficilement utilisables dans les compositions cosmétiques aqueuses et la préparation de compositions comprenant de tels actifs cosmétiques non colorants, doit se faire avec beaucoup de précaution pour éviter le contact de ces composés avec notamment l'humidité, l'oxygène de l'air ou la lumière, avant de les introduire dans une composition cosmétique.

En outre, ces actifs cosmétiques non colorants sensibles à un stimulus extérieur doivent être conditionnés dans des flacons ou récipients étanches relativement coûteux, par exemple, du type flacon-pompe ayant un système sans reprise d'air et/ou un système à poche en matériau opaque.

La Demanderesse a découvert de manière surprenante que l'utilisation d'un nouveau procédé de préparation d'une composition de traitement cosmétique des matières kératiniques comprenant des actifs cosmétiques non colorants sensibles à un stimulus extérieur, permettait de surmonter les problèmes de stabilité exposés ci-dessus. En outre, ce procédé permet d'obtenir en un temps très court, inférieur à 2 minutes, des compositions plus ou moins concentrées en actif(s) cosmétique(s) non colorant(s) sensible(s) à un stimulus extérieur, selon le besoin, notamment sans conservateur.

Ce procédé est mis en oeuvre simplement et est approprié au consommateur. On fait passer un fluide, dont la température est de préférence supérieure ou égale à 30 °C, sous pression, pendant un laps de temps très court, inférieur à 1 minute, à travers au moins un actif cosmétique non colorant sensible à un stimulus extérieur, sous forme solide ou pâteuse, de préférence solide, et encore plus préférentiellement pulvérulente.

Il permet aussi d'utiliser sous forme anhydre ces actifs cosmétiques non colorants notamment instables dans le milieu atmosphérique et/ou dans des compositions aqueuses soit parce qu'ils réagissent avec l'humidité de l'air ou l'eau, soit parce qu'ils réagissent en solution aqueuse avec des composés qui ne réagissent pas avec eux dans une composition anhydre.

Les compositions préparées selon ce procédé peuvent présenter une stabilité au stockage limitée, ce qui n'est pas ici un inconvénient puisque le procédé conduit à une composition prête à l'emploi, destinée à être utilisée rapidement après sa préparation, par exemple dans les 5 minutes suivant sa préparation, notamment après refroidissement jusqu'à une température acceptable pour les matières kératiniques, de préférence inférieure à 60 °C, mieux inférieure à 50 °C. La composition peut être également utilisée jusqu'à une semaine après sa préparation, selon la vitesse de dégradation de l'actif cosmétique non colorant sensible à un stimulus extérieur, utilisé.

Etant donné le temps de préparation très court, les compositions de traitement cosmétique peuvent être préparées "à la demande" en mélangeant différents composés actifs en cosmétique selon les propriétés cosmétiques recherchées.

Selon un autre mode de réalisation, les actifs cosmétiques non colorants sensibles à un stimulus extérieur, pouvant être conditionnés dans un dispositif prêt-à-l'emploi, il n'est pas nécessaire de déterminer au préalable les concentrations desdits actifs en solution, ce qui limite les erreurs de mesure de l'utilisateur.

En outre, le procédé selon l'invention permet d'éviter l'utilisation de flacons étanches multi-compartiments, ce qui rend le procédé particulièrement économique et plus sûr pour l'utilisateur.

La composition ainsi obtenue peut être utilisée seule ou en mélange avec une autre composition.

Un avantage supplémentaire de ce procédé de préparation est l'obtention de compositions présentant de meilleures propriétés cosmétiques. En particulier, les fibres kératiniques traitées avec une composition obtenue par le procédé selon l'invention présentent des propriétés de conditionnement améliorées, et notamment une meilleure protection des cheveux contre les agressions extérieures. La peau quant à elle est ainsi mieux protégée contre le vieillissement, mieux nourrie et hydratée, et une meilleure cicatrisation peut être obtenue.

L'invention a donc pour objet un procédé de préparation d'une composition de traitement cosmétique des matières kératiniques comprenant une étape de percolation d'un fluide sous pression au travers d'au moins un actif cosmétique non colorant sensible à un stimulus extérieur, sous forme solide ou pâteuse.

Un autre objet de l'invention est une composition susceptible d'être obtenue par le procédé selon l'invention.

L'invention a également pour objet l'utilisation de la composition obtenue selon le procédé de l'invention, pour le traitement cosmétique des matières kératiniques, et notamment le conditionnement des cheveux et de la peau.

L'invention a aussi pour objet un procédé de traitement cosmétique des matières kératiniques des êtres humains, comprenant a) la préparation d'une composition cosmétique prête à l'emploi par percolation d'un fluide sous pression d'au moins 3 bars au travers d'au moins un actif cosmétique non colorant sensible à un stimulus extérieur, sous forme solide ou pâteuse, et b) l'application de la composition obtenue à l'étape a) aux matières kératiniques.

L'application aux matières kératiniques peut, selon la nature des actifs, être réalisée par voie topique ou voie orale, et de préférence par voie topique.

L'invention a encore pour objet un dispositif de conditionnement permettant de mettre en oeuvre le procédé de préparation de la présente invention.

D'autres objets, caractéristiques, aspects et avantages de l'invention apparaîtront encore plus clairement à la lecture de la description et des exemples qui suivent.

Selon l'invention, le procédé de préparation d'une composition de traitement cosmétique des matières kératiniques comprend une étape de percolation de fluide, de préférence à une température supérieure ou égale à 30 °C, mieux encore allant de 30 à 150 °C, et encore plus préférentiellement allant de 40 à 120 °C, sous une pression d'au moins 3 bars (3.10⁵ Pa) au travers d'au moins un actif cosmétique non colorant sensible à un stimulus extérieur, sous forme solide ou pâteuse.

La percolation est un mouvement de fluide à travers un milieu poreux permettant le passage du fluide, sous l'action ou l'effet de la pression.

Le fluide comprend au moins de la vapeur d'eau. Il peut être constitué par de la vapeur d'eau éventuellement accompagnée d'eau liquide, ou par un mélange de vapeur d'eau éventuellement accompagnée d'eau liquide, et d'un ou plusieurs solvants liquides et/ou gazeux cosmétiquement acceptables. De préférence, le fluide est de la vapeur d'eau pouvant être accompagnée d'eau liquide.

A titre de solvant organique, on peut citer, par exemple, les alcools inférieurs en C₁-C₄ tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les actifs cosmétiques non colorants sensibles à un stimulus extérieur, utilisés dans l'invention sont sous forme solide ou pâteuse, de préférence sous forme solide, et encore plus préférentiellement pulvérulente.

Par «forme pâteuse» au sens de la présente invention, on entend une consistance intermédiaire entre une phase solide et une phase liquide. La viscosité de cette phase pâteuse est de préférence supérieure à 0,1 Pa.s, et encore plus préférentiellement supérieure à 1 Pa.s, ceci à 25°C avec un taux de cisaillement de 10 s⁻¹.

Par "matières kératiniques", on entend la peau, les paupières, le cuir chevelu, les lèvres, et/ou les phanères tels que les ongles et les fibres kératiniques, par exemple les cils, les sourcils et les cheveux.

Le procédé de la présente invention peut être mis en oeuvre à partir d'un dispositif classique permettant de générer un fluide sous pression, à une température de préférence supérieure ou égale à 30 °C, mieux encore allant de 30 à 150 °C, et encore plus préférentiellement de 40 à 120 °C. Un tel dispositif comprend une chambre résistant à la pression, équipée d'un bloc thermique, ainsi qu'un circuit d'acheminement du fluide vers l'actif cosmétique non colorant sensible à un stimulus extérieur, sous forme solide ou pâteuse.

Selon un autre mode de réalisation, le dispositif comprend de plus un réservoir de liquide(s) ainsi qu'une pompe permettant l'acheminement du ou des liquides vers la chambre.

Le liquide contenu dans le réservoir est soit de l'eau, soit un mélange d'eau et d'un ou plusieurs solvants cosmétiquement acceptables. De préférence, le liquide est de l'eau.

Un dispositif particulièrement utile pour la mise en oeuvre du procédé de la présente invention est une machine à café du type "expresso". De telles machines sont bien connues de la technique. Par exemple, ces machines sont décrites dans les brevets AT 168405, US 2688911, DE 32433870 et IT 1265636.

Selon un mode de réalisation particulier de l'invention, l'étape de percolation est mise en oeuvre avec un fluide à une température supérieure ou égale à 30 °C, de préférence allant de 30 à 150 °C, et encore plus préférentiellement de 40 à 120 °C, sous une pression allant de 3 à 30 bars (3.10⁵ à 3.10⁶ Pa), de préférence d'au moins 4 bars (4.10⁵ Pa), mieux encore supérieure ou égale à 10 bars (10⁶ Pa), et tout particulièrement allant de 10 à 30 bars (10⁶ à 3.10⁶ Pa).

Une composition comprenant au moins un actif cosmétique non colorant, sensible à un stimulus extérieur, sous forme solide ou pâteuse, peut être utilisée directement dans le dispositif générant le fluide sous pression dans un récipient destiné à cet usage. Elle peut aussi être conditionnée dans un dispositif de conditionnement particulier, de type monodose, comprenant un logement fermé délimité par au moins une paroi au moins en partie perméable au fluide sous une pression d'au moins 3 bars. De tels dispositifs sont, par exemple, décrits dans les demandes de brevets WO 00/56629, EP512470, US 5897899 ou WO 99/03573. Ces dispositifs de conditionnement sont en général étanches à l'air, l'humidité et/ou la lumière.

Selon un mode de réalisation particulier, le logement est délimité par deux feuilles scellées. Selon un autre mode de réalisation, le logement est délimité par une barquette fermée par un couvercle.

Ces dispositifs peuvent être fabriqués à partir de matériaux tissés ou non tissés, en matière plastique ou végétale, par exemple en cellulose, en métal tel que l'aluminium ou en composite. De tels dispositifs sont par exemple décrits dans les demandes de brevets WO 00/56629, EP512470, US 5897899 ou WO 99/03573.

Les actifs cosmétiques non colorants, sensibles à un stimulus extérieur, utilisables dans la présente invention peuvent être des actifs cosmétiques non colorants, sensibles à l'eau, à l'oxydation et/ou à la lumière, et de préférence à l'eau. A titre d'exemples de tels actifs, on peut notamment citer les vitamines, les enzymes, des extraits naturels, l'urée, des composés antioxydants ou antiradicalaires, certains acides, des acides gras polyinsaturés ou des filtres solaires.

Comme actifs cosmétiques non colorants sensibles à l'eau et à l'oxydation, utilisables dans la présente invention, on peut notamment citer :
- les vitamines telles que l'acide ascorbique (vitamine C) et ses dérivés, notamment ses dérivés phosphatés comme le sel de potassium du di-alpha-tocopheryl-di-ascorbyl-phosphate (vendu par la Société Senju Pharmaceutical sous la référence SEPIVITAL EPC), l'ascorbylphosphate de magnésium et l'ascorbylphosphate de sodium (vendu par la Société Roche sous la référence Stay-C 50), et ses esters comme l'acétate, le palmitate et le propionate d'ascorbyle ; le rétinol (vitamine A) et ses dérivés, notamment ses esters comme l'acétate, le palmitate et le propionate de rétinol ; l'acide pantothénique (vitamine B) et ses dérivés comme la pantolactone, le D-panthénol ; la biotine (vitamine H), la phytodione (vitamine K1) qui peut être utilisée pour le traitement des télangiectasies de la peau, et en particulier la couperose ;
- les enzymes, par exemple, une lactoperoxydase, une lipase, une protéase, une phospholipase, une cellulase ou une superoxydedismutase ;
- les extraits naturels sous forme solide tels qu'un extrait de mélisse, un extrait de thym, des oligomères procyannidoliques (ou OPC) tels que l'OPC d'aubépine, l'OPC de pin (pycnogénols) et l'OPC de raisin, les extraits de fruits comme les extraits de cassis et de myrtille ;
- l'urée ;
- les flavonoïdes tels que la rutine destinée à être utilisée pour l'amincissement et le contour des yeux ;
- le sulfure de sélénium utilisé notamment comme agent antipelliculaire,
- certains acides tels que l'acide kojique, l'acide rétinoique et ses dérivés tels que le rétinol et le palmitate de rétinol, l'acide benzène-1,4-di-(3-méthylidène-10-camphosulfonique), l'acide salicylique et ses dérivés comme l'acide n-octanoyl-5- salicylique ;
- les acides gras polyinsaturés tels que l'acide gamma-linolénique ou linoléique et leurs esters.

Les actifs cosmétiques non colorants sensibles à la lumière, utilisables dans la présente invention peuvent être certaines vitamines comme la phytodione (vitamine K1), des flavonoïdes, des rétinoïdes ou l'anthraline.

Les actifs cosmétiques non colorants sensibles à un stimulus extérieur particulièrement préférés dans la présente invention sont les enzymes, l'urée, les flavonoïdes, le sulfure de sélénium, les acides tels que décrits ci-dessus et les acides gras polyinsaturés.

Les actifs cosmétiques non colorants sensibles à un stimulus extérieur, peuvent être mis en oeuvre en mélange avec un ou plusieurs adjuvants solides ou pâteux, et de préférence pulvérulents. Les adjuvants peuvent être choisis parmi les argiles, les sels, les tensioactifs anioniques, non ioniques, cationiques ou zwittérioniques, les épaississants naturels ou de synthèse, l'amidon éventuellement modifié, les billes de verre, la silice, le nylon, l'alumine, le dioxyde de titane, les zéolithes, le poly(méthacrylate de méthyle) (PMMA), le chitosane, la maltodextrine, la cyclodextrine, les mono- ou disaccharides comme le glucose, le saccharose, le sorbitol et le fructose, l'oxyde de zinc, de zirconium, les particules de résine comme la silicone ou les silicabades, le talc, l'acide polyaspartique, les borosilicates notamment de calcium, le polyéthylène, le coton, le polytétrafluoroéthylène (PTFE), la cellulose et ses dérivés, les composés superabsorbants, les carbonates de magnésium ou de calcium, les grains de maïs, les gommes de polydiméthylsiloxane, le polyacrylamide, l'hydroxyapatite poreux, la soie, le collagène, la sciure de bois, la poudre de fucus, les farines ou extraits de blé, de riz, de pois, de lupin, de soja ou d'orge, la polyvinylpyrrolidone réticulée, l'alginate de calcium, le charbon actif, les particules de poly(chlorure de vinylidène/acrylonitrile), notamment celles commercialisées sous la dénomination générale d' « Expancel® » par la société AKZO NOBEL sous les références particulières « Expancel® WE» ou « DE » Expancels, et leurs mélanges.

Lorsqu'un ou plusieurs adjuvants sont présents, ledit ou lesdits actifs cosmétiques non colorants sensibles à un stimulus extérieur, sont présents de préférence en une quantité allant de 0,5 à 99% en poids, mieux encore de 1 à 80 % en poids, et encore plus préférentiellement de 2 à 60 % en poids par rapport au poids total actif(s) cosmétique(s) non colorant(s) sensible(s) à un stimulus extérieur, et adjuvant(s) sous forme solide ou pâteuse.

La composition de traitement cosmétique des matières kératiniques obtenue selon le procédé de l'invention contient outre le(s) actif(s) cosmétique(s) non colorant(s) sensible(s) à un stimulus extérieur et le(s) composant(s) du fluide, à savoir l'eau et/ou le(s) solvant(s) cosmétiquement acceptable(s), éventuellement tout ou partie du ou des adjuvants présents dans le mélange sous forme solide ou pâteuse.

Lorsque des plantes ou extraits de plantes sont utilisés dans le procédé selon l'invention, ils peuvent être soumis, avant la percolation, à un traitement tel qu'une torréfaction, un cryobroyage, ou une lyophilisation.

L'invention concerne également une composition susceptible d'être obtenue par le procédé selon l'invention, la composition particulièrement préférée ne comprenant aucun agent conservateur.

A partir du procédé de préparation de l'invention, on obtient une composition de traitement cosmétique des matières kératiniques qui peut être appliquée directement sur les matières kératiniques, ou qui peut être mélangée à un milieu cosmétiquement acceptable, ou encore au moins un additif classiquement utilisé en cosmétique pourra y être ajouté par un opérateur. On peut également mélanger au moins deux compositions obtenues par le procédé de l'invention. La composition de traitement cosmétique des matières kératiniques résultant éventuellement de mélange(s) et/ou addition(s) indiqués ci-dessus, sera dénommée ci-après composition finale de traitement cosmétique ou composition finale.

Un mode de réalisation particulier de l'invention consiste à appliquer la composition obtenue par l'intermédiaire d'un dispositif ne nécessitant pas d'intervention humaine et équipé éventuellement d'un moyen de refroidissement.

Un autre mode de réalisation particulier consiste à ingérer la composition de traitement cosmétique obtenue selon le procédé de l'invention lorsque aucun problème de toxicité n'est connue dans le technique, par exemple lorsque ledit actif cosmétique est un acide gras, une vitamine telle que les vitamines A et E.

La quantité dudit ou desdits actifs cosmétiques non colorants sensibles à un stimulus extérieur, présents dans la composition finale de traitement cosmétique est en général comprise dans l'intervalle allant de 0,001 à 50% en poids environ, de préférence de 0,005 à 30% en poids, et encore plus préférentiellement de 0,01 à 20% en poids du poids total de la composition finale de traitement cosmétique.

Lorsque la composition de traitement cosmétique obtenue par le procédé de la présente invention est mélangée à un milieu cosmétiquement acceptable, un tel milieu est généralement constitué par de l'eau ou par un mélange d'eau et d'au moins un solvant organique pour solubiliser les composés qui ne seraient pas suffisamment solubles dans l'eau.

Par "cosmétiquement acceptable", on entend un milieu compatible avec les matières kératiniques et notamment la peau, les lèvres et/ou les phanères, et qui en outre, présente un aspect, un toucher, une odeur et éventuellement un goût agréable pour l'utilisateur.

A titre de solvant organique, on peut citer, par exemple, les alcools inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 40 % en poids, et encore plus préférentiellement entre 5 et 30 % en poids par rapport au poids total de la composition finale de traitement cosmétique.

Au moins un additif classiquement utilisé en cosmétique peut être également ajouté dans les compositions de traitement cosmétique obtenues selon le procédé de la présente invention. A titre d'exemples de tels additifs, on peut citer des agents tensioactifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement différents de ceux décrits ci-dessus tels que, par exemple, des huiles de silicone, des agents filmogènes, des céramides, des agents conservateurs, des agents opacifiants, des pigments nacrés ou colorés.

Les additifs ci-dessus sont en général présents en une quantité comprise pour chacun d'eux entre 0,01 et 20 % en poids par rapport au poids de la composition finale.

Bien entendu, l'homme de l'art veillera à choisir ce ou ces éventuels additifs de manière telle que les propriétés avantageuses attachées intrinsèquement à la composition de traitement cosmétique conforme à l'invention ne soient pas, ou substantiellement pas, altérées par la ou les adjonctions envisagées.

Le pH de la composition finale de traitement cosmétique est généralement compris entre 3 et 12, et de préférence entre 5 et 11. Il peut être ajusté à la valeur désirée au moyen d'agents acidifiants ou alcalinisants habituellement utilisés en cosmétique ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants, on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule suivante : 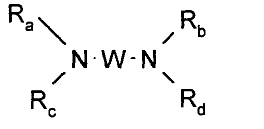 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

La composition finale de traitement cosmétique peut se présenter sous des formes diverses, telles que sous forme de liquides, de crèmes, de gels ou sous toute autre forme appropriée pour être utilisée comme shampoing, après-shampoing, soin rincé ou non rincé, masque de soin profond, gel douche, lotion ou crème de traitement des matières kératiniques, ou produit de protection solaire de la peau ou des cheveux.

La présente invention concerne aussi un procédé de traitement cosmétique des matières kératiniques, comprenant la préparation d'une composition de traitement cosmétique selon le procédé tel que défini ci-dessus, et son application sur les matières kératiniques, par exemple par l'intermédiaire d'un opérateur ou par l'intermédiaire d'un dispositif ne nécessitant pas d'intervention humaine. La durée d'application peut varier entre 15 secondes et 1 heure.

Avant application, la composition de traitement cosmétique obtenue selon le procédé de l'invention, peut être mélangée à un milieu cosmétiquement acceptable et/ou à un ou plusieurs additifs classiquement utilisés en cosmétique tels que décrits ci-dessus.

Un autre mode de réalisation consiste à préparer au moins deux compositions de traitement cosmétique selon le procédé de l'invention, à les mélanger, et à ajouter éventuellement un milieu cosmétiquement acceptable et/ou un ou plusieurs additifs classiquement utilisés en cosmétique tels que décrits ci-dessus, puis à appliquer la composition finale obtenue sur les matières kératiniques.

L'exemple ci-après illustre la présente invention.

### EXEMPLES

### Exemple 1

On mélange les ingrédients solides suivants dans les proportions indiquées en % en poids par rapport au poids total de mélange solide :
- Urée pure 50 %
- Lauroylamidopropylbétaïne en poudre vendue sous la dénomination commerciale Amphotensid B4 PV 25 %
- Saccharose en poudre 25 %

On place 5 g. de ce mélange dans une machine expresso du commerce. On fait ensuite passer de la vapeur d'eau jusqu'à l'obtention d'une composition (A) présentant un volume final de 50 ml.

On obtient une composition de traitement prête à être appliquée sur la peau.

Après application, la peau présente une bonne hydratation.

On peut ajouter à deux parties en poids de composition (A), une partie en poids d'une composition aqueuse (B) contenant 1 % en poids d'hydroxyéthylcellulose pour faciliter l'application.

### Exemple 2

On place une poudre de protéase dans une machine expresso du commerce. On fait ensuite passer de la vapeur d'eau jusqu'à l'obtention d'une composition (A) présentant un volume final de 50 ml.

On obtient une composition prête à être appliquée sur le visage. Elle permet de lisser le visage et d'éclaircir le teint.

On peut ajouter à une partie en poids de composition (A), une partie en poids d'une composition aqueuse (B) contenant 2 % en poids d'alginate de propylèneglycol estérifié à 80-85 % pour faciliter l'application.

## Revendications

1. Procédé de préparation d'une composition de traitement cosmétique des matières kératiniques, **caractérisé en ce qu'**il comprend une étape de percolation de fluide comprenant au moins de la vapeur d'eau, sous une pression d'au moins 3 bars au travers d'au moins un actif cosmétique non colorant sensible à un stimulus extérieur, sous forme solide ou pâteuse.

2. Procédé selon la revendication 1, **caractérisé en ce que** le fluide est constitué par de la vapeur d'eau éventuellement accompagnée d'eau liquide, ou encore par un mélange de vapeur d'eau éventuellement accompagnée d'eau liquide, et d'un ou plusieurs solvants liquides et/ou gazeux cosmétiquement acceptables.

3. Procédé selon la revendication 1 ou 2, **caractérisé en ce que** l'actif cosmétique non colorant sensible à un stimulus extérieur est choisi parmi les vitamines, les enzymes, des extraits naturels, l'urée, les composés antixoydants ou antiradicalaires, l'acide kojique, l'acide rétinoique et ses dérivés, l'acide benzène-1,4-di-(3-méthylidène-10-camphosulfonique), l'acide salicylique et ses dérivés, des acides gras polyinsaturés, le sulfure de sélénium et des filtres solaires.

4. Procédé selon la revendication 3, **caractérisé en ce que** ledit actif cosmétique est choisi parmi les enzymes, l'urée, les flavonoïdes, le sulfure de sélénium, l'acide kojique, l'acide rétinoique et ses dérivés, l'acide benzène-1,4-di-(3-méthylidène-10-camphosulfonique), l'acide salicylique et ses dérivés, et les acides gras polyinsaturés.

5. Procédé selon la revendication 4, **caractérisé en ce que** les enzymes sont choisies parmi une lactoperoxydase, une lipase, une protéase, une phospholipase, une cellulase et une superoxydedismutase.

6. Procédé selon la revendication 4, **caractérisé en ce que** le flavonoïde est la rutine.

7. Procédé selon la revendication 4, **caractérisé en ce que** les acides gras polyinsaturés sont choisis parmi l'acide gamma-linolénique ou linoléique et leurs esters.

8. Procédé selon l'une quelconque des revendications précédentes, **caractérisé en ce que** l'actif cosmétique non colorant sensible à un stimulus extérieur, sous forme solide ou pâteuse est mis en oeuvre en mélange avec au moins un adjuvant.

9. Procédé selon la revendication 8, **caractérisé en ce que** l'adjuvant est choisi parmi les argiles, les sels, les tensioactifs anioniques, non ioniques, cationiques ou zwittérioniques, les épaississants naturels ou de synthèse, l'amidon éventuellement modifié, les billes de verre, la silice, le nylon, l'alumine, le dioxyde de titane, les zéolithes, le poly(méthacrylate de méthyle) (PMMA), le chitosane, la maltodextrine, la cyclodextrine, les mono- ou disaccharides, l'oxyde de zinc, de zirconium, les silicabades, le talc, l'acide polyarpartique, les borosilicates notamment de calcium, le polyéthylène, le coton, le polytétrafluoroéthylène (PTFE), la cellulose, les composés superabsorbants, les carbonates de magnésium ou de calcium, les grains de maïs, les gommes de polydiméthylsiloxane, le polyacrylamide, l'hydroxyapatite poreux, la soie, le collagène, la sciure de bois, la poudre de fucus, les farines ou extraits de blé, de riz, de pois, de lupin, de soja ou d'orge, la polyvinylpyrrolidone réticulée, l'alginate de calcium, le charbon actif, et les particules de poly(chlorure de vinylidène/acrylonitrile).

10. Procédé selon la revendication 8 ou 9, **caractérisé en ce que** le ou les actifs cosmétiques non colorants sensibles à un stimulus extérieur, sont présents en une quantité allant de 0,5 à 99% en poids, de préférence de 1 à 80 % en poids par rapport au poids total actif(s) cosmétique(s) non colorant(s) sensible(s) à un stimulus extérieur et adjuvant(s) sous forme solide ou pâteuse.

11. Procédé selon l'une quelconque des revendications précédentes, **caractérisé en ce que** la composition de traitement cosmétique obtenue contient outre le(s) actif(s) cosmétique(s) non colorant(s) sensible(s) à un stimulus extérieur et le(s) composant(s) du fluide, éventuellement tout ou partie du ou des adjuvants présents dans le mélange sous forme solide ou pâteuse.

12. Procédé selon l'une quelconque des revendications précédentes, **caractérisé en ce que** l'étape de percolation est mise en oeuvre avec un fluide sous pression allant de 3 à 30 bars.

13. Procédé selon la revendication 12, **caractérisé en ce que** l'étape de percolation est mise en oeuvre avec un fluide sous pression d'au moins 10 bars.

14. Composition de traitement cosmétique des matières kératiniques susceptible d'être obtenue par le procédé selon l'une quelconque des revendications précédentes.

15. Composition de traitement cosmétique des matières kératiniques selon la revendication 14, ne comprenant pas de conservateur.

16. Procédé de traitement cosmétique des matières kératiniques, **caractérisé en ce que** l'on prépare une composition de traitement cosmétique selon le procédé défini selon l'une quelconque des revendications 1 à 13, et que l'on applique cette composition sur les matières kératiniques.

17. Procédé de traitement cosmétique des matières kératiniques selon la revendication 16, **caractérisé en ce que** l'on applique cette composition sur les matières kératiniques par l'intermédiaire d'un dispositif ne nécessitant pas d'intervention humaine.

18. Procédé de traitement cosmétique des matières kératiniques selon la revendication 16, **caractérisé en ce que**, avant l'application, la composition de traitement cosmétique préparée selon le procédé selon l'une quelconque des revendications 1 à 13, est mélangée à un milieu cosmétiquement acceptable et/ou à un ou plusieurs additifs utilisés en cosmétique.

19. Procédé de traitement cosmétique des matières kératiniques, **caractérisé en ce que** l'on prépare au moins deux compositions de traitement cosmétique selon le procédé défini selon l'une quelconque des revendications 1 à 13, on les mélange et on applique le mélange sur les matières kératiniques.

20. Dispositif de conditionnement d'une composition cosmétique, comprenant un logement fermé délimité par au moins une paroi au moins en partie perméable au fluide sous pression d'au moins 3 bars, ladite composition contenant au moins un actif cosmétique non colorant sensible à un stimulus extérieur, sous forme solide ou pâteuse.

21. Dispositif selon la revendication 20, dans lequel le logement est délimité par deux feuilles scellées.

22. Dispositif selon la revendication 20, dans lequel le logement est délimité par une barquette fermée par un couvercle.

23. Utilisation de la composition obtenue par le procédé selon l'une quelconque des revendications 1 à 19, pour le traitement cosmétique des matières kératiniques.

24. Utilisation de la composition obtenue par le procédé selon la revendication 23, pour le conditionnement des cheveux ou de la peau.
